# EUROPEAN PATENT APPLICATION

(11) **EP 3 070 161 A1**
(43) Date of publication of application: **21.09.2016**
(21) Application number: 16160469.9
(22) Date of filing: 15.03.2016
(51) Int. Cl.: C12N 5/00, C12N 5/0789, C12N 5/0775

(54) **METHOD OF PREPARING SOLUTION CONTAINING STEM CELLS**

(30) Priority: 16.03.2015 US 201562133478 P; 21.09.2015 US 201514859392
(71) Applicant: Stembios Technologies, Inc., Monterey Park CA 91754 (US)
(72) Inventor: Wang, James, Monterey Park, CA 91754 (US)
(74) Representative: Wittmann, Günther

(57) **Abstract**

A method of preparing a stem-cell containing solution includes: (1) storing a mixture of a blood sample and a divalent cation chelating agent at a temperature between 2 and 12 degrees Celsius for a period of from 3 hours to 72 hours so as to have the mixture with two or more separate layers, wherein a liquid in one of the separate layers contains platelets and stem cells; (2) collecting the liquid into a container; (3) centrifuging the liquid in the container until multiple particles suspended in the liquid are deposited at a bottom of the container, wherein the particles contain the stem cells and the platelets; and (4) re-suspending the particles in a buffer or liquid.

## Description

### BACKGROUND OF THE DISCLOSURE

This application claims priority to U.S. provisional application No. 62/133,478, filed on Mar. 16, 2015 and claims priority to US patent application No. 14859392, filed on Sept. 21, 2015, all of which are incorporated herein by references in their entirety.

### Field of the Disclosure

The disclosure relates to a method of preparing a solution containing cells, and more particularly, to a method of preparing a solution containing (somatic) stem cells.

### Brief Description of the Related Art

Stem cells have the ability to self-renew to generate more stem cells and also to become almost any type of specialized cells. Stem cell research is useful for learning about human development and is one of the most fascinating areas of contemporary biology. Therefore, stem cells offer exciting promise for future medical science.

### SUMMARY OF THE DISCLOSURE

An exemplary embodiment of the present disclosure provides a method of forming or preparing a stem-cell containing solution. The method may include: (1) storing a mixture of a blood sample and a divalent cation chelating agent (e.g., K2 EDTA or K3 EDTA) at a temperature between 2 and 12 degrees Celsius (°C), and more preferably between 2 and 7 °C, or of about 4 °C, for a period of from 3 hours to 72 hours, from 3 hours to 6 hours, from 6 hours to 72 hours, from 6 hours to 48 hours, from 16 hours to 72 hours, from 16 hours to 48 hours, from 36 hours to 60 hours, or from 48 hours to 72 hours, so as to have the mixture with two or more separate layers (e.g., including an upper layer and a lower layer), wherein a first liquid (e.g., a supernatant liquid) in one of the separate layers (e.g., the upper layer or the topmost one of the separate layers) contains multiple platelets (which, for example, are less than 6 micrometers in size) and multiple somatic stem cells (e.g., including CD349(+) (pluripotent) somatic stem cells and Lgr5(+) (pluripotent) somatic stem cells); (2) collecting the first liquid from the mixture; and (3) after collecting the first liquid from the mixture, centrifuging the first liquid so as to have multiple pellets or particles gather in the first liquid, wherein the pellets or particles contain the somatic stem cells and the platelets. The method may further include: after the step of centrifuging the first liquid, removing a first portion of the first liquid and leaving the pellets or particles gathering in a second portion of the first liquid, and then re-suspending the pellets or particles in the second portion of the first liquid so as to prepare or form the stem-cell containing solution. Alternatively, the method may further include: after the step of centrifuging the first liquid, removing substantially all or a portion of the first liquid and leaving the pellets or particles, and then re-suspending the pellets or particles in a second liquid so as to prepare or form the stem-cell containing solution. The second liquid may be or may contain a salt solution such as (Dulbecco's) phosphate-buffered saline or a solution free from Ca²⁺ (which is further free from any other divalent ions, including Mg²⁺). The solution free from Ca²⁺, for example, is normal saline, 0.9% salt solution. Alternatively, the second liquid may contain the salt solution and another portion of the first liquid. The second liquid contains and is mixed with the pellets or particles after the step of re-suspending the pellets or particles in the second liquid. The stem-cell containing solution, for example, is employed or used for a dental implant treatment or a bone graft procedure.

Another exemplary embodiment of the present disclosure provides a method of preparing a stem-cell containing solution, which, for example, is employed or used for a dental implant treatment or a bone graft procedure. The method includes: (1) incubating a blood sample with a divalent cation chelating agent (e.g., K2 EDTA or K3 EDTA) at a temperature between 2 and 12 degrees Celsius (°C), and more preferably between 2 and 7 °C, or of about 4 °C, for a time period, such as between 3 hours and 72 hours, and more preferably between 3 hours and 6 hours, between 6 hours and 72 hours, between 6 hours and 48 hours, between 16 hours and 72 hours, between 16 hours and 48 hours, between 36 hours and 60 hours, or between 48 hours and 72 hours, so that the blood sample is formed with multiple separate layers (e.g., including an upper layer and a lower layer); (2) collecting one of the separate layers (e.g., the upper layer) into a container (e.g., a tube), wherein said one of the separate layers contains multiple platelets (which, for example, are less than 6 micrometers in size) and multiple somatic stem cells (which, for example, contain CD349(+) somatic stem cells and Lgr5(+) somatic stem cells); (3) centrifuging said one of the separate layers in the container until multiple pellets or particles suspended in said one of the separate layers are deposited at/on a bottom of the container, wherein the pellets or particles contain the somatic stem cells and the platelets; and (4) re-suspending the deposited pellets or particles in a liquid solution so as to prepare or form the stem-cell containing solution. The liquid solution may be or may include a salt solution such as (Dulbecco's) phosphate-buffered saline or a solution free from Ca²⁺ (which may be further free from any other divalent ions, including Mg²⁺). The solution free from Ca²⁺, for example, is normal saline, 0.9% salt solution. Alternatively, the liquid solution may contain the salt solution and a portion of said one of the separate layers. Alternatively, the liquid solution may be a portion of said one of the separate layers. The liquid solution contains and is mixed with the pellets or particles after the step of re-suspending the deposited pellets or particles in the liquid solution.

In the above-mentioned methods, the somatic stem cells, for example, are between 1 and 6 micrometers in size, and more preferably greater than 1 or 2 micrometers and less than 6 micrometers in size. The somatic stem cells may also contain CD66e(+) somatic stem cells, i.e., blastomere-like stem cells (BLSCs), which may be greater than 2 micrometers and less than 6 micrometers in size. The CD349(+) somatic stem cells are also CD9(+) and, for example, are greater than 2 micrometers and less than 6 micrometers in size. The Lgr5(+) somatic stem cells are also Oct4(+) and Nanog(+), as well as CD133(-), CD66e(-), Sox2(-), CD4(-), CD8(-), CD9(-), CD10(-), CD11(-), CD16(-), CD17(-), CD18(-), CD19(-), CD20(-), CD21(-), CD31(-), CD42(-), CD63(-), CD34(-), Lin(-), CD38(-), CD90(-), CD45(-), and CD349(-). The Lgr5(+) somatic stem cells, for example, are greater than 2 micrometers and less than 6 micrometers in size. The percentage of the Lgr5(+) somatic stem cells in cells greater than 2 micrometers and less than 6 micrometers in size in the stem-cell containing solution or the pellets or particles is greater than 4% or 5%, and more preferably between 4.5% and 10%. The percentage of the CD349(+) somatic stem cells in cells greater than 2 micrometers and less than 6 micrometers in size in the stem-cell containing solution or the pellets or particles is greater than 4% or 5%, and more preferably between 4.5% and 10%. Less than 1% of cells in the stem-cell containing solution or the pellets or particles may express CD133. The percentage of white blood cells in cells greater than 2 micrometers and less than 20 micrometers in size in the stem-cell containing solution or the pellets or particles is less than 2%, and more preferably less than 1%, 0.5% or 0.1%. The percentage of red blood cells in cells greater than 2 micrometers and less than 10 micrometers in size in the stem-cell containing solution or the pellets or particles is less than 3%, and more preferably less than 2%, 1% or 0.5%. The blood sample may be derived from the peripheral blood of a subject and contains a plurality of cells. The subject is a human or an animal.

An exemplary embodiment of the present disclosure provides a stem-cell containing solution for a dental implant treatment or a bone graft procedure. The stem-cell containing solution contains CD349(+) somatic stem cells (SB-1 cells) that are greater than 2 micrometers and less than 6 micrometers in size, Lgr5(+) somatic stem cells (SB-2 cells) that are greater than 2 micrometers and less than 6 micrometers in size, and CD66e(+) somatic stem cells (blastomere-like stem cells) that are greater than 2 micrometers and less than 6 micrometers in size. The stem-cell containing solution may further contain a salt (such as sodium chloride) or a salt solution. The salt solution, for example, is (Dulbecco's) phosphate-buffered saline or a solution free from Ca²⁺ (which may be further free from any other divalent ions, including Mg²⁺). The solution free from Ca²⁺, for example, is normal saline, 0.9% salt solution. The percentage of the Lgr5(+) somatic stem cells in cells greater than 2 micrometers and less than 6 micrometers in size in the stem-cell containing solution is greater than 4% or 5%, and more preferably between 4.5% and 10%. The percentage of the CD349(+) somatic stem cells in cells greater than 2 micrometers and less than 6 micrometers in size in the stem-cell containing solution is greater than 4% or 5%, and more preferably between 4.5% and 10%. The percentage of the CD66e(+) somatic stem cells in cells less than 6 micrometers and greater than 2 micrometers in size in the stem-cell containing solution is less than 6%, and more preferably less than 5% or 4.5%. The percentage of white blood cells in cells greater than 2 micrometers and less than 20 micrometers in size in the stem-cell containing solution is less than 2%, and more preferably less than 1%, 0.5% or 0.1%. The percentage of red blood cells in cells greater than 2 micrometers and less than 10 micrometers in size in the stem-cell containing solution is less than 3%, and more preferably less than 2%, 1% or 0.5%.

These, as well as other steps, features, benefits, and advantages of the present disclosure, will now become clear from a review of the following detailed description of illustrative embodiments, the accompanying drawings, and the claims.

### BRIEF DESCRIPTION OF THE DRAWINGS

Aspects of the disclosure may be more fully understood from the following description when read together with the accompanying drawings, which are to be regarded as illustrative in nature, and not as limiting. In the drawings:
Fig. 1 shows a flow chart illustrating a method of forming or preparing a stem-cell containing solution according to an embodiment of the present disclosure;
Fig. 2 shows a flow chart illustrating a method of forming or preparing a stem-cell containing solution according to an embodiment of the present disclosure;
Fig. 3 shows the content/ingredient information of a brown algae fucoidan supplement;
Fig. 4 shows that stem-cell data of six human subjects are obtained before and after the oral ingestion of a brown algae fucoidan supplement;
Figs. 5A and 5B show that flow cytometry data are obtained before and after a course of injections with granulocyte stimulating factor (GCSF or G-CSF);
Fig. 6A is a forward scattering coefficient (FSC) versus side scattering coefficient (SSC) flow cytometry dot plot;
Fig. 6B is a fluorescence histogram from flow cytometry analysis;
Fig. 6C is a FSC versus SSC flow cytometry dot plot;
Fig. 7A is a FSC versus SSC flow cytometry dot plot; and
Figs. 7B-7G are fluorescence histograms from flow cytometry analysis.

While certain embodiments are depicted in the drawings, one skilled in the art will appreciate that the embodiments depicted are illustrative and that variations of those shown, as well as other embodiments described herein, may be envisioned and practiced within the scope of the present disclosure.

### DETAILED DESCRIPTION OF THE INVENTION

Illustrative embodiments are now described. Other embodiments may be used in addition or instead. Details that may be apparent or unnecessary may be omitted to save space or for a more effective presentation. Conversely, some embodiments may be practiced without all of the details that are disclosed.

Before describing embodiments of the present invention, a definition or description has been included for these various terms. These definitions or descriptions are provided to assist in teaching a general understanding of the present invention.

### Definition of size (Z) of a cell:

The size (Z) of a cell such as (somatic) stem cell or biological cell, mentioned in all following paragraphs, of the present disclosure may be, but not limited to, described or defined as (1) the conventional definition of the size or representative length of a cell in the field of cell biology or the field of stem cells, (2) the diameter of a cell especially when the cell is substantially spherical, (3) the length of the major axis of a cell especially when the cell is substantially ellipsoidal, (4) the width of a cell when the shape of the cell has an approximate shape of a square, (5) the length of a cell when the shape of the cell has an approximate shape of a rectangle, or (6) the greatest cross-sectional or transverse dimension of a cell. The size (Z), either the diameter, length, width, or greatest cross-sectional or transverse dimension, can be, but not limited to, determined or measured, for example, using an image of the cell obtained from an optical microscope or from an electron microscope such as a scanning electron microscope (SEM), or using data (e.g., two-dimensional dot, contour or density plot) of the cell obtained from a flow cytometer. The image of the cell obtained from the optical microscope or electron microscope may be a two-dimensional (2D) cross section or three-dimensional (3D) structure of the cell. As an example, the size (Z) of the cell may be obtained by, e.g., measuring the greatest cross-sectional or transverse dimension of the cell in a 2D cross-sectional image obtained from an optical microscope or an electron microscope (e.g., SEM).

### Description of stem cells:

Somatic stem cells (also called adult stem cells) can be found in an organ or tissue such as bone marrow, fat or (peripheral) blood and possess the same basic characteristics of all stem cells. A somatic stem cell is an unspecialized or undifferentiated cell, which is capable of differentiation into specialized cell types. In the present disclosure, somatic stem cells are not embryonic stem cells; in other words, the somatic stem cells are not derived, sourced or harvested from embryos or fetal tissue.

There are various types of somatic stem cells, including totipotent stem cells, pluripotent stem cells, multipotent stem cells, and progenitor stem cells (also called unipotent stem cells). Blastomere-like stem cells (BLSCs) are totipotent or pluripotent stem cells. Very small embryonic-like stem cells (VSELs) are pluripotent somatic stem cells. SB-1 cells and SB-2 cells are pluripotent or multipotent somatic stem cells. Mesenchymal stem cells (MSCs), hematopoietic stem cells (HSCs), multipotent adult progenitor cells (MAPCs), bone marrow derived multipotent stem cells (BMSCs), and multipotent adult stem cells (MASCs) are multipotent somatic stem cells. Neural stem cells, retina stem cells, olfactory bulbs stem cells, epidermal stem cells, muscle stem cells, intestine stem cells, pancreatic stem cells, heart stem cells, liver stem cells, kidney stem cells, endothelial stem cells, adipocyte or adipose-derived stem cells, marrow-isolated adult multilineage inducible (MIAMI) cells, pre-mesenchymal stem cells (pre-MSCs), mesenchymal progenitor cells, hematopoietic progenitor cells (HPCs), multipotent progenitor cells (MPPs), lineage-restricted progenitor cells (LRPs), common myeloid progenitor cells (CMPs), and common lymphocyte progenitor cells (CLPs) are progenitor stem cells.

In the following paragraphs the sign "+" following a cell (surface) marker means cells (e.g., stem cells) can express the cell (surface) marker; in the other term, the cell (surface) marker existing in the cell surfaces of the cells may be detected by performing a flow cytometry using a (marker-specific) antibody. And, the sign "-" following a cell (surface) marker means cells (e.g., stem cells) do not express the cell (surface) marker; in the other term, the cell (surface) marker, not existing in the cell surfaces of the cells, may not be detected by performing a flow cytometry using a (marker-specific) antibody. The positive sign "+" is a positive expression of a cell (surface) marker for a cell, and the negative sign "-" is a negative expression of a cell (surface) marker for a cell.

A SB-1 cell, which may be a pluripotent or multipotent somatic stem cell having a nucleus, is a CD349(+) cell. The CD349(+) somatic stem cell, i.e., the SB-1 cell, may be also CD9(+), Oct4(+), and Nanog(+), as well as CD133(-), CD90(-), CD34(-), and Sox2(-). The CD349(+) somatic stem cell, for example, is smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers, or between 1.0 and 4.0 micrometers, in size (as defined by the above-mentioned size (Z) of a cell). Preferably, the SB-1 cell is greater than 2 micrometers and less than 6 micrometers in size. The SB-1 cell can express the cell (surface) marker CD349 and can be characterized by CD349(+).

A SB-2 cell, which may be a pluripotent or multipotent somatic stem cell having a nucleus, is a Lgr5(+) cell. The Lgr5(+) somatic stem cell, i.e., the SB-2 cell, may be also Oct4(+) and Nanog(+), as well as CD133(-), CD66e(-), CD4(-), CD8(-), CD9(-), CD10(-), CD11(-), CD16(-), CD17(-), CD18(-), CD19(-), CD20(-), CD21(-), CD31(-), CD42(-), CD63(-), CD34(-), Lin(-), CD38(-), CD90(-), CD45(-), CD349(-), and Sox2(-). The Lgr5(+) somatic stem cell, for example, is smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Preferably, the Lgr5(+) somatic stem cell is greater than 2 micrometers and less than 6 micrometers in size. The SB-2 cell can express the cell (surface) marker Lgr5 and can be characterized by Lgr5(+). In the present disclosure, SB cells contain CD349(+) somatic stem cells (SB-1 cells) and Lgr5(+) somatic stem cells (SB-2 cells).

A blastomere-like stem cell (BLSC), which may be a totipotent or pluripotent somatic stem cell having a nucleus, is a CD66e(+) cell. The CD66e(+) somatic stem cell, i.e., the BLSC, may be smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Preferably, BLSC is greater than 2 micrometers and less than 6 micrometers in size. The BLSC can express the cell (surface) marker CD66e and can be characterized by CD66e(+).

Very small embryonic-like stem cells (VSELs), which may be pluripotent somatic stem cells each having a nucleus, contain CD133(+) somatic stem cells and CD34(+) somatic stem cells. The CD133(+) somatic stem cells and the CD34(+) somatic stem cells, i.e., the VSELs, are also CD45(-) and Lin(-). The CD133(+) somatic stem cells and the CD34(+) somatic stem cells, for example, are smaller (or less) than or equal to 4, 5 or 6 micrometers, such as between 0.1 and 6.0 micrometers, between 0.5 and 6.0 micrometers, between 1.0 and 6.0 micrometers, between 2.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). Preferably, the CD133(+) somatic stem cells and the CD34(+) somatic stem cells are greater than 2 micrometers and less than 6 micrometers in size. In the case of the CD133(+), CD45(-), Lin(-) somatic stem cell, the VSEL can express the cell (surface) marker CD133 and lacks expression of the two cell (surface) markers CD45 and Lin and can be characterized by CD133(+), CD45(-), and Lin(-). In the case of the CD34(+), CD45(-), Lin(-) somatic stem cell, the VSEL can express the cell (surface) marker CD34 and lacks expression of the two cell (surface) markers CD45 and Lin and can be characterized by CD34(+), CD45(-), and Lin(-).

A mesenchymal stem cell (MSC), which may be a multipotent somatic stem cell having a nucleus, may be a CD13(+), CD29(+), CD44(+), CD73(+), CD90(+) or CD105(+) multipotent stem cell. The MSC may express one or more of the cell (surface) markers CD 13, CD29, CD44, CD73, CD90 and CD105 and may be characterized by one or more of CD13(+), CD29(+), CD44(+), CD73(+), CD90(+) and CD105(+). Mesenchymal stem cells (MSCs) are very heterogeneous populations which mean to have various sizes and shapes. Some types of MSCs may be smaller (or less) than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). The other types of MSCs may be greater than 6, 7 or 10 micrometers in size (as defined by the above-mentioned size (Z) of a cell).

Hematopoietic stem cells (HSCs), which may be multipotent somatic stem cells each having a nucleus, contain CD34(+), cKit(-), CD38(-), Lin(-) cells and CD150(+), CD244(-), CD48(-) cells. The CD34(+), cKit(-), CD38(-), Lin(-) somatic stem cells and the CD150(+), CD244(-), CD48(-) somatic stem cell, i.e., the HSCs, may be smaller (or less) than or equal to 4, 5 or 6 micrometers, such as between 0.1 and 6.0 micrometers, between 0.5 and 6.0 micrometers, between 1.0 and 6.0 micrometers, between 0.1 and 5.0 micrometers, between 0.5 and 5.0 micrometers, between 1.0 and 5.0 micrometers, between 0.1 and 4.0 micrometers, between 0.5 and 4.0 micrometers or between 1.0 and 4.0 micrometers, in size (as defined by the above-mentioned size (Z) of a cell). In the case of the CD34(+), cKit(-), CD38(-), Lin(-) somatic stem cell, the HSC can express the cell (surface) marker CD34 and lacks expression of the cell (surface) markers cKit, CD38 and Lin and can be characterized by CD34(+), cKit(-), Lin(-) and CD38(-). In the case of the CD150(+), CD244(-), CD48(-) somatic stem cell, the HSC can express the cell (surface) marker CD150 and lacks expression of the two cell (surface) markers CD244 and CD48 and can be characterized by CD150(+), CD244(-) and CD48(-).

### Description of actions (X):

The following items are examples of the actions (X), which may be previously evaluated effective in increasing the number of cells for a type or selected types of stem cells in vivo in a subject such as human body or entity or non-human body or entity. The non-human body or entity may be an animal body or entity. The actions (X) include:
1. Taking drugs such as synthetic drugs or drugs including extractions from nature;
2. Taking herbs or Chinese or herbal medicines, such as Cordyceps sinensis, ginseng, Lycium Chinense Mill, Ganoderma lucidum (lingzhi), Taiwanofungus camphoratus, and/or Brazil mushroom;
3. Taking nutrients or dietary supplements, such as nutrition pills or powder, including the following materials or elements: vitamins (Vitamin A, B, B complex, B₁₂, D, D₃, E, etc.), macro and/or trace minerals (e.g., calcium, sodium, potassium, fluorine, bromine, chromium, iodine, silicon, selenium, beryllium, lithium, cobalt, vanadium and/or nickel), polysaccharides, high molecular weight fucose-containing glycoproteins, seaweed (including green algae, blue-green algae, brown algae, and etc.), fucose, fucoidan that is a major component of brown algae, oligo fucoidan, algae, brown algae containing fucoidan (for example, brown algae grown and produced in Okinawa, Japan), Japanese Mozuku, green algae, blue-green algae (or blue algae), brown algae (including mozuku, kelp, undaria, sargassum fusiforme, pinnatifida, and etc.), phytochemical (e.g., isoflavones or phytoestrogen), lycopene, epigallocatechin gallate (EGCG), green tea essence, gluconutrients (e.g., Xylose, Galactose, Glucose, Mannose N-acetylglucosamine, N-acetylgalaetosanmine, or N-acetylneuraminic acid), fish oil, China toona (toona sinensis), and/or nutrients extracted from plant, leaf, fruit, vegetable, fish, seaweed, or algae;
4. Practicing a vegetarian dietary;
5. Taking or eating healthy food or organic food;
6. Taking alternative (non-traditional) medicine;
7. Being subjected to alternative therapy or treatment such as the Gerson therapy or the Breuss cancer cure;
8. Being subjected to acupuncture;
9. Being subjected to massage such as foot massage;
10. Exercising such as walking, jogging, dancing, gymnastics, Yoga, aerobic exercise, and/or Taijiquan (Chinese shadow exercise);
11. Sleeping (for purpose of measuring the quality of sleep);
12. Meditating;
13. Exercising a health improvement program or a disease curing program designed by an individual, a health professional, or a medical doctor;
14. Taking a certain nutrient for improving health of a certain organ in a body, for example, taking lycopene to improve the health of prostate;
15. Taking a rehabilitation program to heal the injury, or to heal the wounds caused by surgery, or to cure a disease;
16. Taking a medicinal liquor (or called medicinal wine, medicated liquor or medicated wine) made from, e.g., immersing one Chinese medicine or multiple Chinese medicines in liquor or wine for a period of time, such as ginseng wine made from immersing ginseng in a high alcohol concentration rice wine for a month;
17. Taking one or more drugs approved by a government department or authority, such as U.S. food and drug administration (U.S. FDA), for curing a specific disease (e.g., a type of cancer, skin disease, kidney disease and/or so on);
18. Taking or being subjected to treatments or therapies approved by a government department for curing a specific disease (e.g., a type of cancer, skin disease, or kidney disease);
19. Exercising or participating a religious activity, such as praying for peace or worshiping God;
20. Being exposed directly or indirectly to sunshine or sunlight (in the morning between, for example, 10 minutes before sunrise and 50 minutes after sunrise (containing significant amount of infrared (IR) light); or around noon, for example, between 11:30 AM to 12:30 PM (containing significant amount of ultra-violet (UV) light); or in the afternoon, for example, between 50 minutes before sunset and 10 minutes after sunset (containing significant amount of infrared (IR) light));
21. Being exposed to the lamp light or the light emitting diode (LED) light, which may include a whole spectrum of visible lights, IR light, red light, green light, blue light, or UV light, or a combination of more than one of the above lights;
22. Exercising or being subjected to programs, therapies, methods, apparatus and/or systems for improving body's self-healing, for example, a method or therapy (e.g., Hyperbaric oxygen therapy) performed after injury or surgery for improving self-healing;
23. Drinking coffee such as black coffee;
24. Drinking tea such green tea, black tea, or jasmine tea;
25. Drinking red wine;
26. Taking melatonin;
27. Listening to music such as Mozart's or Beethoven's symphony;
28. Injecting a substance (e.g., nutrient or supplement) containing fucoidan or oligo fucoidan;
29. Taking hormone supplements or being subjected to a hormone injection;
30. Injecting a granulocyte-colony stimulating factor (G-CSF or GCSF), which is a cytokine or hormone (glycoprotein) that can stimulate the bone marrow to produce granulocytes and stem cells and release them into the bloodstream;
31. Being subjected to a course of GCSF injections; and
32. Taking a nutrient, a nutrient product, a nutrient fluid, a nutrient drink, a nutrient liquid, or a nutrient food containing (1) varieties of amino acids (such as Arginine, Histidine, Lysine, Aspartic acid, Glutamic acid, Serine, Threonine, Asparagine, Glutamine, Cysteine, Valine, Proline, Glycine, Selenocysteine, Alanine, Isoleucine, Leucine, Phenylalanine, Methionine, Tyrosine, or Tryptophan), (2) balanced amino acids, or (3) 9 essential amino acids (i.e., Histidine, Isoleucine, Leucine, Lysine, Methionine, Phenylalanine, Threonine, Tryptophan and Valine) for human bodies. For examples: (a) Product produced or extracted from the fermentation of red, green, black beans; (b) Liquid, fluid, or drink produced from fermentation of a fruit or a combination of fruits, such as sugar beet, apple, guava, kiwi, grape, pineapple, red pitaya (dragon fruit), green papaya, tomato, and/or avocado, etc.; (c) A medicinal liquor (or called medicinal wine, medicated liquor, or medicated wine) made from, e.g., immersing one Chinese medicine or multiple Chinese medicines in liquor or wine for a period of time, such as ginseng wine made from immersing ginseng in a high alcohol concentration rice wine for a month.

### Embodiments:

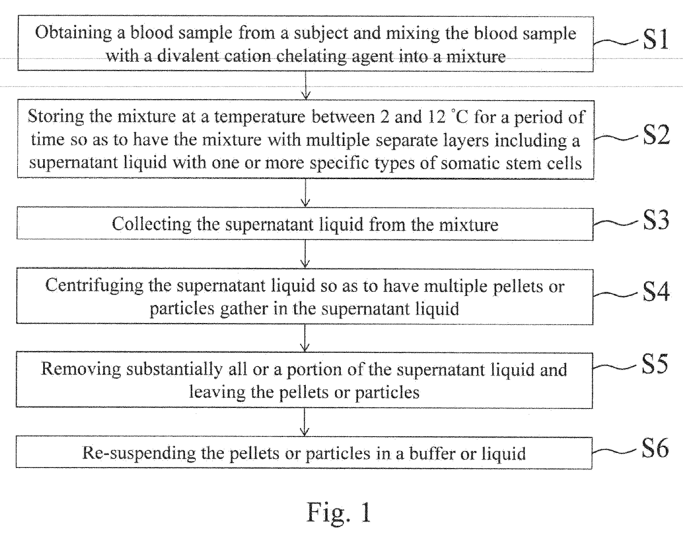

A stem-cell containing solution is prepared, obtained or formed by sequentially performing six steps S1 through S6 illustrated in Fig. 1. Referring to Fig. 1, in the step S1, a blood sample is extracted, drawn, taken, obtained, collected or derived from the peripheral blood of a subject, a non-embryonic tissue of the subject, into one or more first containers (e.g., a bag, one or more syringes, or one or more tubes) containing a divalent cation chelating agent, and mixed with the divalent cation chelating agent well so that a first mixture of the blood sample and the divalent cation chelating agent is formed in the one or more first containers. The divalent cation chelating agent, an anticoagulant, may be ethylenediaminetetraacetic acid (EDTA), such as K2 EDTA anticoagulant or K3 EDTA anticoagulant, having a weight, e.g., greater than 70 mg, such as between 90 and 900 mg, between 120 and 450 mg, or between 150 and 400 mg. Alternatively, the divalent cation chelating agent may be citrate having a weight, e.g., greater than 70 mg, such as between 90 and 900 mg, between 120 and 450 mg, or between 150 and 400 mg. The subject, for example, is a human (e.g., child, teenager, adult, or graybeard) or an animal (e.g., mammal). Examples of the animal are listed as below: primate (e.g., monkey or gorilla), dog, rodent (e.g., mouse or guinea pig), cat, horse, cow, cattle, sheep, pig, chicken, duck, goose, bird, and elephant.

The blood sample contains a plurality of cells, including small cells less than and equal to 6 micrometers in size and large cells greater than 6 micrometers in size. The small cells, for example, contain platelets (which, for example, are less than 6 micrometers in size) and small somatic stem cells (which are less than and equal to 6 micrometers in size, and more preferably greater than 1 or 2 micrometers in size). For instance, the small somatic stem cells contain one or more specific types of somatic stem cells (e.g., SB cells), BLSCs (i.e., CD66e(+) somatic stem cells), and VSELs (e.g., CD133(+) somatic stem cells and CD34(+) somatic stem cells). The large cells, for example, contain large somatic stem cells greater than 6 micrometers in size and lineage cells containing red blood cells and white blood cells. The blood sample may have a volume greater than or equal to 20, 30 or 45 milliliters, such as between 20 and 250 milliliters, between 60 and 500 milliliters, between 80 and 250 milliliters, or between 100 and 200 milliliters. To obtain or form the first mixture, the blood sample, for example, may be mixed with 1.5 mg or more, such as between 1.6 and 2.0 mg, of the divalent cation chelating agent (such as K2 EDTA, K3 EDTA, or citrate) per milliliter of the blood sample. The one or more specific types of somatic stem cells, for example, may be or may include one or more types of stem cells described in the section of the "Description of stem cells". For instance, the one or more specific types of somatic stem cells may be or may include somatic stem cells having a size equal to or less than 6 micrometers (e.g., between 1 and 6 micrometers, or greater than 1 or 2 micrometers and less than 6 micrometers), such as SB-1 cells, SB-2 cells, BLSCs, VSELs, MSCs, and/or HSCs. Alternatively, the one or more specific types of somatic stem cells may be or may include somatic stem cells less than 6 micrometers in size, and more preferably greater than 2 micrometers in size, such as CD349(+) somatic stem cells (i.e., SB-1 cells) and/or Lgr5(+) somatic stem cells (i.e., SB-2 cells) and/or CD66e(+) somatic stem cells (i.e., BLSCs).

In the step S2, the first mixture, for example, is stored at a temperature between 2 degrees Celsius (°C) and 12 °C, and more preferably between 2 °C and 7 °C, or of about 4 °C, in a suitable facility (e.g., a refrigerator or other device used to keep things cold) for a (predetermined) period of time, such as between 3 hours and 72 hours, and more preferably between 3 hours and 6 hours, between 6 hours and 72 hours, between 6 hours and 48 hours, between 16 hours and 72 hours, between 16 hours and 48 hours, between 36 hours and 60 hours, or between 48 hours and 72 hours, or around 48 hours. After the first mixture has been stored for the (predetermined) period of time, the one or more specific types of somatic stem cells (e.g., SB cells) in the first mixture are activated by the divalent cation chelating agent (such as K2 EDTA, K3 EDTA, or citrate), i.e., the cell cycle of the one or more specific types of somatic stem cells is activated from G0 into G1. The activation relates to the ability of the divalent cation chelating agent to repress p53's function (presumably by chelating Zn²⁺), thereby allowing the one or more specific types of somatic stem cells to turn from the G0 quiescence stage into the cell cycle G1. As the p53 protein requires Zn²⁺ to fold properly and form a functional protein, chelating Zn²⁺ by the divalent cation chelating agent would be a key step to activate the one or more specific types of somatic stem cells. It is possible that the divalent cation chelating agent can chelate other divalent ions (e.g., Ca²⁺) and thereby activates the one or more specific types of somatic stem cells that are in G0 phase.

After having been stored at the temperature for the (predetermined) period of time, the first mixture is formed with two or more separate layers (which, for example, include an upper layer and a lower layer) because of gravity. One of the separate layers (such as the upper layer or the topmost one of the separate layers), for example, is a supernatant liquid (which may have a volume between 6 and 120 milliliters, between 20 and 250 milliliters, between 40 and 125 milliliters, or between 50 and 100 milliliters) and can be categorized into three parts - platelets (which, for example, are less than 6 micrometers in size), serum, and the small somatic stem cells containing, e.g., the one or more specific types of somatic stem cells (e.g., SB cells), BLSCs (i.e., CD66e(+) somatic stem cells), and VSELs (e.g., CD133(+) somatic stem cells and CD34(+) somatic stem cells. The supernatant liquid in said one of the separate layers may also include the divalent cation chelating agent (e.g., EDTA) and/or growth factors. Another one of the separate layers such us the lower layer has most of the large cells containing the lineage cells (such as red and white blood cells) and the large somatic stem cells in the first mixture. For example, said another one of the separate layers may have greater than 95%, 98%, 99% or more of the large cells in the first mixture. Accordingly, the supernatant liquid substantially excludes the large cells (such as red blood cells and white blood cells) or may include less than 0.5, 1, 2 or 5 percent of the large cells in the first mixture. The supernatant liquid, for example, may include less than 1 or 2 percent of red blood cells in the first mixture and less than 1 or 2 percent of white blood cells in the first mixture; at least 98 or 99 percent of red blood cells in the first mixture and at least 98 or 99 percent of white blood cells in the first mixture may gather in said another one of the separate layers (e.g., the lower layer). The ratio value of the volume of the supernatant liquid to the volume of the blood sample, for example, may range from one third to one half. The ratio value of the volume of the supernatant liquid to the volume of the first mixture may range from one third to one half. In summary, in accordance with the steps S1 and S2, the blood sample from the subject is incubated with the divalent cation chelating agent at the temperature for the (predetermined) period of time so that the blood sample is formed with the separate layers including the supernatant liquid.

In the step S3, substantially all of the supernatant liquid may be collected or transferred into a second container, such as a bag, a glass bottle, or a syringe. The supernatant liquid in the second container (hereinafter the "cell mixture") contains the small cells, such as platelets and the small somatic stem cells containing, e.g., the one or more specific types of somatic stem cells (e.g., SB cells), BLSCs (i.e., CD66e(+) somatic stem cells), and VSELs (e.g., CD133(+) somatic stem cells and CD34(+) somatic stem cells. The number of somatic stem cells greater than 2 micrometers and less than 6 micrometers in size in the cell mixture, for example, is greater than or equal to 10 million or 30 million, and more preferably between 25 million and 300 million, between 30 million and 500 million, or between 10 million and 500 million. The cell mixture may also contain the divalent cation chelating agent (e.g., EDTA) and/or growth factors. The percentage of the number of the large cells in the cell mixture to the number of total particles or cells in the cell mixture may be less than 5%, 1%, or 0.5%, and more preferably less than 0.1% or 0.01%; the percentage of the number of the small cells in the cell mixture to the number of the total particles or cells in the cell mixture may be greater than 95%, 99%, or 99.5%, and more preferably less than 99.9% or 99.99%.

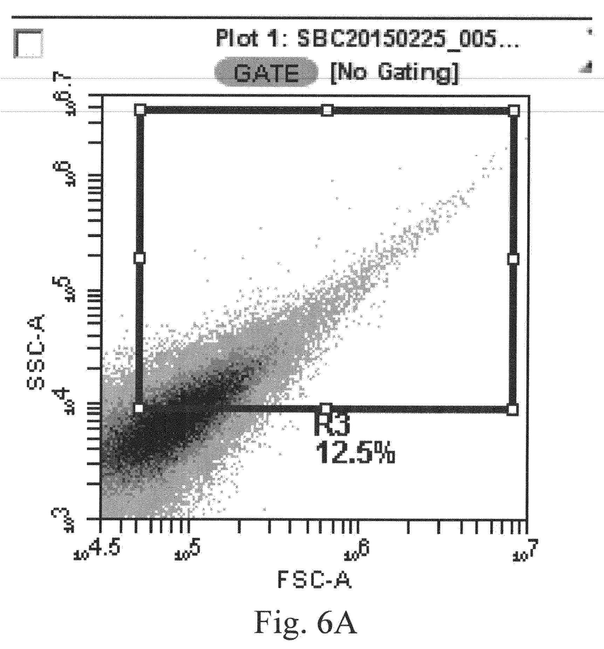

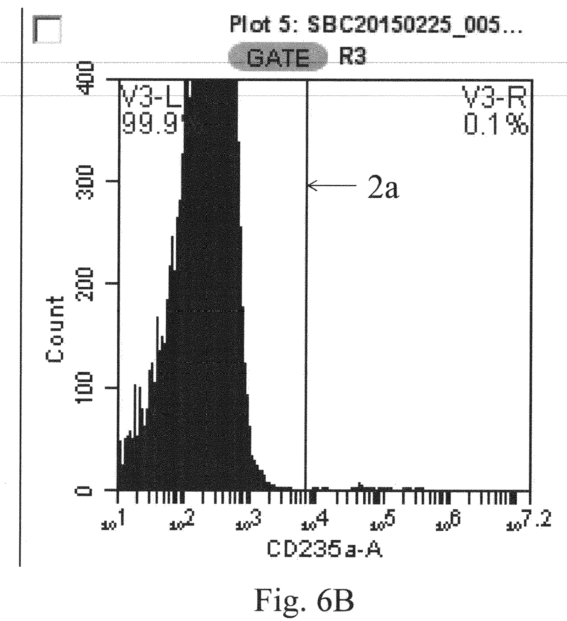

The number of red blood cells per milliliter of the cell mixture is calculated based on, e.g., data of Figs. 6A and 6B. Fig. 6A is obtained by analyzing 10 microliters (µl) of the cell mixture with a flow cytometer. Red blood cells (i.e., CD235a(+) cells) are in a region R3 of Fig. 6A; the number of all cells in the region R3 of Fig. 6A is 25000. Fig. 6B shows the distribution of CD235a fluorescence intensity in all cells in the region R3 of Fig. 6A. In Fig. 6B, the fluorescence histogram is separated into a region V3-L with low fluorescence intensity and a region V3-R with high fluorescence intensity by a vertical line 2a (i.e., reference). The region V3-R of Fig. 6B represents cells stained positive for CD235a, i.e., red blood cells; the region V3-L of Fig. 6B represents cells stained negative for CD235a. The result of Fig. 6B indicates that the percentage of the number of red blood cells in the region R3 of Fig. 6A to the number of all cells in the region R3 of Fig. 6A is 0.1%. By multiplying the number of all cells in the region R3, i.e., 25000, by the percentage of the number of red blood cells in the region R3 of Fig. 6A to the number of all cells in the region R3 of Fig. 6A, i.e., 0.1%, the number of red blood cells in the 10 microliters of the cell mixture is calculated and found to be equal to 25. Therefore, the number of red blood cells per milliliter of the cell mixture is calculated from the number of red blood cells in the 10 microliters of the cell mixture and found to be equal to 2500. It can be seen from the above result that the number of red blood cells per milliliter of the cell mixture can be less than 10⁵ or 10⁴. Preferably, the number of red blood cells per milliliter of the cell mixture is less than 10³. In addition, the percentage of red blood cells in cells greater than 2 micrometers and less than 10 micrometers in size in the cell mixture may be less than 3%, and more preferably less than 2%, 1% or 0.5%.

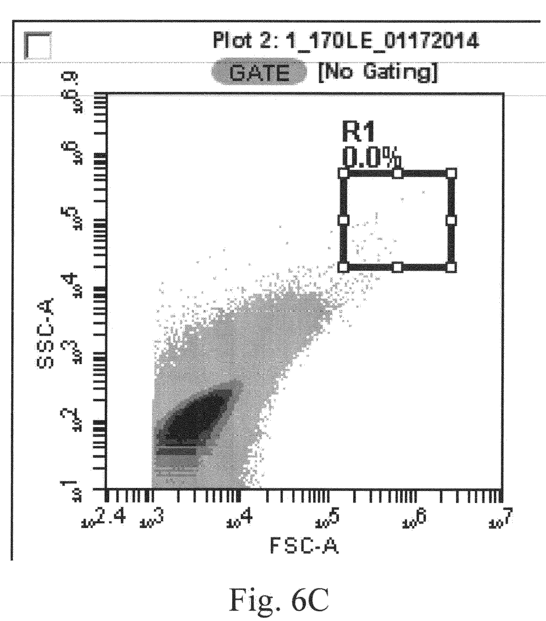

The number of white blood cells per milliliter of the cell mixture is calculated based on, e.g., flow cytometry data in Fig. 6C. The flow cytometry data in Fig. 6C are obtained by analyzing 50 microliters (µl) of the cell mixture with a flow cytometer. In Fig. 6C, a region R1 is white-blood-cell (WBC) gating. Based on the flow cytometry data in Fig. 6C, the number of white blood cells in the 50 microliters of the cell mixture is calculated and found to be equal to 30. Therefore, the number of white blood cells per milliliter of the cell mixture is calculated from the number of white blood cells in the 50 microliters of the cell mixture and found to be equal to 600. It can be seen from the above result that the number of white blood cells per milliliter of the cell mixture can be less than 10⁴ or 10³. Preferably, the number of white blood cells per milliliter of the cell mixture is less than 10². In addition, the percentage of white blood cells in cells greater than 2 micrometers and less than 20 micrometers in size in the cell mixture may be less than 2%, and more preferably less than 1%, 0.5% or 0.1%.

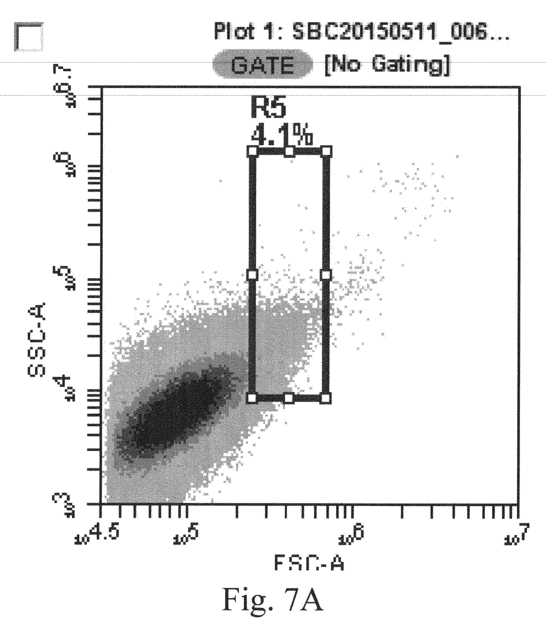

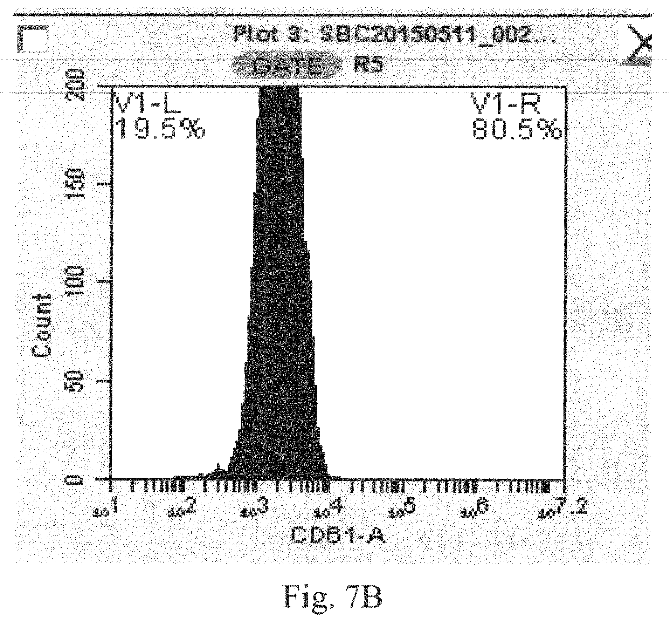

Fig. 7A shows a forward scattering (FSC) versus side scattering (SSC0) flow cytometry dot plot, which is obtained by analyzing 5.6 microliters (µl) of the cell mixture with a flow cytometer. In Fig. 7A, a region R5 represents cells that are less than 6 micrometers and greater than 2 micrometers in size. In other words, all cells in the region R5 are greater than 2 micrometers and less than 6 micrometers in size. Fig. 7B shows the distribution of CD61 fluorescence intensity in all cells in the region R5 of Fig. 7A. In Fig. 7B, the fluorescence histogram is separated into a region V1-L with low fluorescence intensity and a region V1-R with high fluorescence intensity by a vertical line (i.e., reference). The region V1-R of Fig. 7B represents cells stained positive for CD61, i.e., platelets; the region V1-L of Fig. 7B represents cells stained negative for CD61. The result of Fig. 7B indicates that the percentage of the number of platelets in the region R5 to the number of all cells in the region R5 is 80.5%. Therefore, the percentage of platelets in cells less than 6 micrometers and greater than 2 micrometers in size in the cell mixture can be greater than 75% or 80%, and more preferably between 75% and 85%.

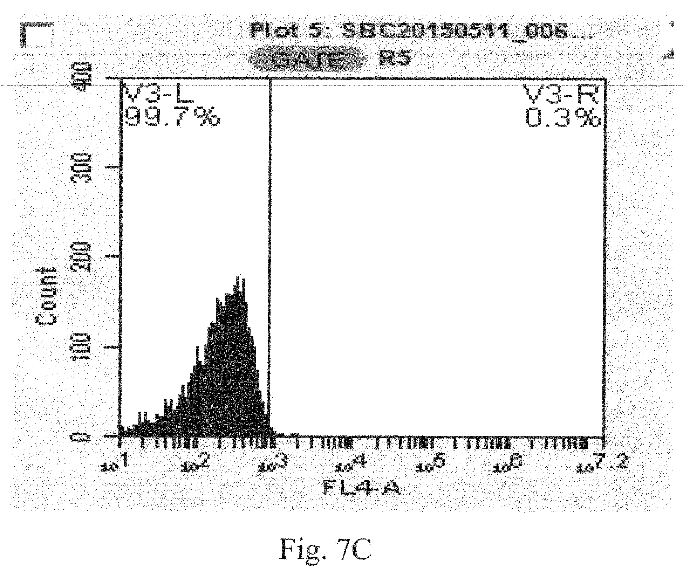

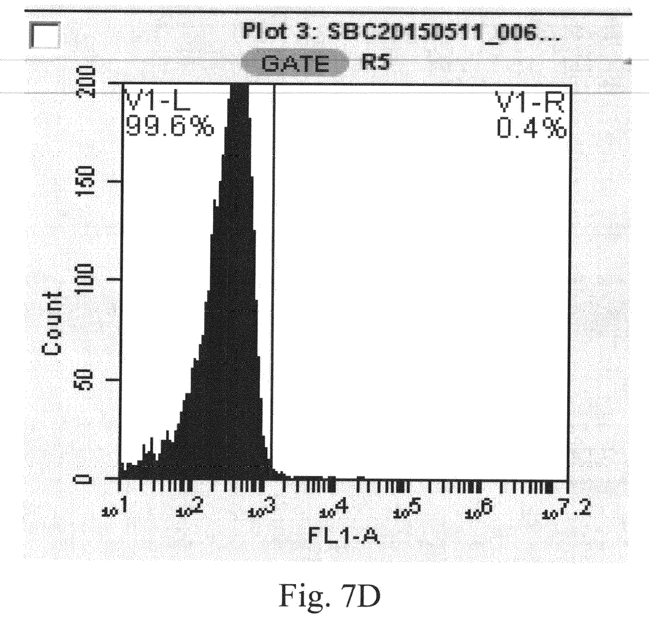

Fig. 7C shows the distribution of CD 133 fluorescence intensity in all cells in the region R5 of Fig. 7A. In Fig. 7C, the fluorescence histogram is separated into a region V3-L with low fluorescence intensity and a region V3-R with high fluorescence intensity by a vertical line (i.e., reference). The region V3-R of Fig. 7C represents cells stained positive for CD133, i.e., VSELs; the region V3-L of Fig. 7C represents cells stained negative for CD133. The result of Fig. 7C indicates that the percentage of the number of CD133(+) cells in the region R5 to the number of all cells in the region R5 is 0.3%. It can be seen from the result that less than 1% of cells in the cell mixture express CD133. Fig. 7D shows the distribution of CD34 fluorescence intensity in all cells in the region R5 of Fig. 7A. In Fig. 7D, the fluorescence histogram is separated into a region V1-L with low fluorescence intensity and a region V1-R with high fluorescence intensity by a vertical line (i.e., reference). The region V1-R of Fig. 7D represents cells stained positive for CD34, i.e., VSELs; the region V1-L of Fig. 7D represents cells stained negative for CD34. The result of Fig. 7D indicates that the percentage of the number of CD34(+) cells in the region R5 to the number of all cells in the region R5 is 0.4%. Therefore, the percentage of the combination of CD133(+) cells and CD34(+) cells in cells less than 6 micrometers and greater than 2 micrometers in size in the cell mixture can be less than 2%, and more preferably less than 1% or 0.5%.

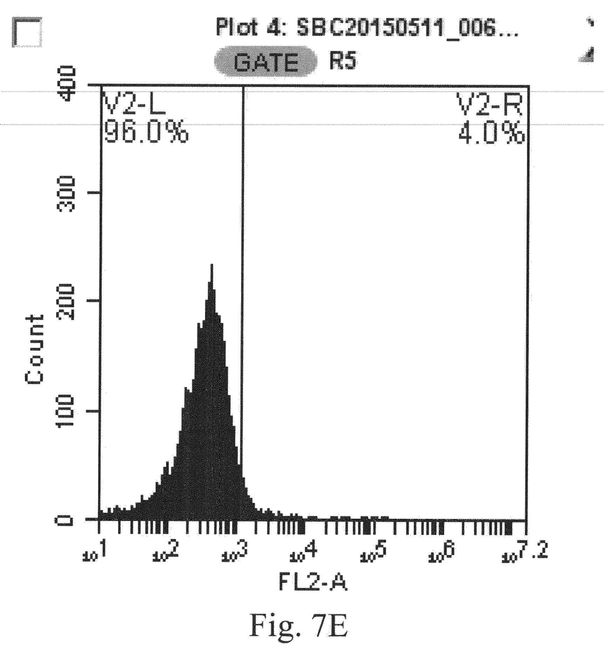

Fig. 7E shows the distribution of CD66e fluorescence intensity in all cells in the region R5 of Fig. 7A. In Fig. 7E, the fluorescence histogram is separated into a region V2-L with low fluorescence intensity and a region V2-R with high fluorescence intensity by a vertical line (i.e., reference). The region V2-R of Fig. 7E represents cells stained positive for CD66e, i.e., BLSCs; the region V2-L of Fig. 7E represents cells stained negative for CD66e. The result of Fig. 7E indicates that the percentage of the number of CD66e(+) cells in the region R5 to the number of all cells in the region R5 is 4%. Therefore, the percentage of CD66e(+) cells in cells less than 6 micrometers and greater than 2 micrometers in size in the cell mixture can be less than 6%, and more preferably less than 5% or 4.5%.

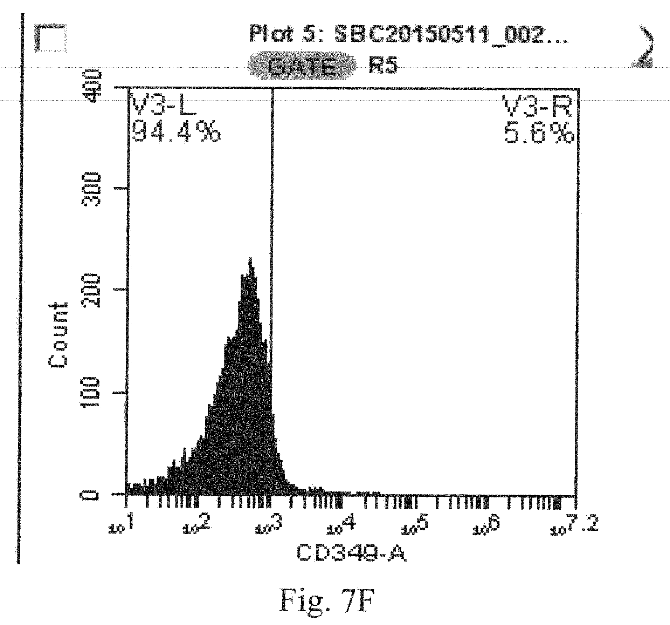

Fig. 7F shows the distribution of CD349 fluorescence intensity in all cells in the region R5 of Fig. 7A. In Fig. 7F, the fluorescence histogram is separated into a region V3-L with low fluorescence intensity and a region V3-R with high fluorescence intensity by a vertical line (i.e., reference). The region V3-R of Fig. 7F represents cells stained positive for CD349, i.e., SB-1 cells; the region V3-L of Fig. 7F represents cells stained negative for CD349. The result of Fig. 7F indicates that the percentage of the number of CD349(+) cells in the region R5 to the number of all cells in the region R5 is 5.6%. Therefore, the percentage of CD349(+) cells in cells less than 6 micrometers and greater than 2 micrometers in size in the cell mixture can be greater than 4% or 5%, and more preferably between 4.5% and 10%.

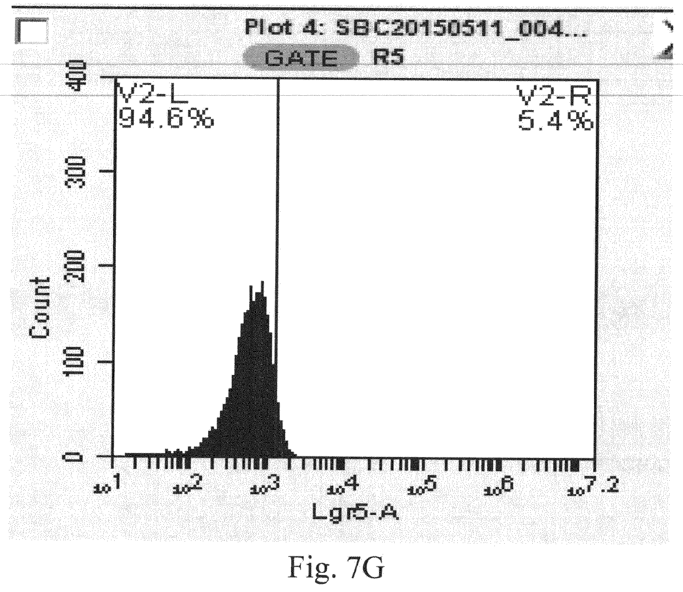

Fig. 7G shows the distribution of Lgr5 fluorescence intensity in all cells in the region R5 of Fig. 7A. In Fig. 7G, the fluorescence histogram is separated into a region V2-L with low fluorescence intensity and a region V2-R with high fluorescence intensity by a vertical line (i.e., reference). The region V2-R of Fig. 7G represents cells stained positive for Lgr5, i.e., SB-2 cells; the region V2-L of Fig. 7G represents cells stained negative for Lgr5. The result of Fig. 7G indicates that the percentage of the number of Lgr5(+) cells in the region R5 to the number of all cells in the region R5 is 5.4%. Therefore, the percentage of Lgr5(+) cells in cells less than 6 micrometers and greater than 2 micrometers in size in the cell mixture solution can be greater than 4% or 5%, and more preferably between 4.5% and 10%.

In the step S4, the cell mixture, i.e., the supernatant liquid in the second container, is centrifuged at a suitable rotational speed (e.g., ranging from, equal to or greater than 1,200 revolutions per minute [rpm] up to, equal to or less than 3,000 rpm, and more preferably, from, equal to or greater than 1,400 rpm up to, equal to or less than 2,000 rpm) for a suitable time period (e.g., ranging from, equal to or greater than 10 minutes up to, equal to or less than 25 minutes, and more preferably, from, equal to or greater than 15 minutes up to, equal to or less than 20 minutes) so that multiple pellets or particles suspended in the cell mixture are deposited or gather at a bottom of the second container. In other words, after centrifuged, the cell mixture can be generalized or classified into two major parts - a centrifuged liquid and the pellets or particles gathering or deposited at the bottom of the second container, in the centrifuged liquid in the second container, and at a bottom of the centrifuged liquid in the second container. The pellets or particles contain the small cells, such as platelets and the small somatic stem cells containing, e.g., the one or more specific types of somatic stem cells (e.g., SB cells), BLSCs (i.e., CD66e(+) somatic stem cells), and VSELs (e.g., CD133(+) somatic stem cells and CD34(+) somatic stem cells).

According to the results from Figs. 6A-6C and 7A-7G, it can be estimated, assumed or presumed that the percentage of SB-1 cells in cells greater than 2 micrometers and less than 6 micrometers in size in the pellets or particles may be greater than 4% or 5%, and more preferably between 4.5% and 10%; the percentage of SB-2 cells in cells greater than 2 micrometers and less than 6 micrometers in size in the pellets or particles may be greater than 4% or 5%, and more preferably between 4.5% and 10%; the percentage of BLSCs in cells less than 6 micrometers and greater than 2 micrometers in size in the pellets or particles may be less than 6%, and more preferably less than 5% or 4.5%; the percentage of VSELs in cells less than 6 micrometers and greater than 2 micrometers in size in the pellets or particles may be less than 2%, and more preferably less than 1% or 0.5%; less than 1% of cells in the pellets or particles may express CD133; the percentage of platelets in cells less than 6 micrometers and greater than 2 micrometers in size in the pellets or particles may be greater than 75% or 80%, and more preferably between 75% and 85%; the percentage of white blood cells in cells greater than 2 micrometers and less than 20 micrometers in size in the pellets or particles may be less than 2%, and more preferably less than 1%, 0.5% or 0.1%; the percentage of red blood cells in cells greater than 2 micrometers and less than 10 micrometers in size in the pellets or particles may be less than 3%, and more preferably less than 2%, 1% or 0.5%.

In the step S5, almost all or a top portion of the centrifuged liquid may be removed from the second container but the pellets or particles deposited or gathering at the bottom of the second container, in the centrifuged liquid, and at the bottom of the centrifuged liquid are left in the second container or in a bottom portion of the centrifuged liquid remaining in the second container. In the step S6, an appropriate amount of a buffer or liquid may be added into the second container, and then the pellets or particles, left in the second container in the step S5, are re-suspended in a liquid solution (which is or contains the buffer or liquid) into a stem-cell containing solution (or called a stem-cell mixture), which thereby includes the pellets or particles, such as platelets and the small somatic stem cells containing, e.g., the one or more specific types of somatic stem cells (e.g., SB cells), BLSCs (i.e., CD66e(+) somatic stem cells), and VSELs (e.g., CD133(+) somatic stem cells and CD34(+) somatic stem cells). Alternatively, the liquid solution may further contain the bottom portion of the centrifuged liquid. The liquid solution contains and is mixed with the pellets or particles after the step of re-suspending the deposited pellets or particles in the liquid solution. The buffer or liquid may be or may include phosphate-buffered saline (such as Dulbecco's phosphate-buffered saline) or a medium or solution free from Ca²⁺. The medium or solution free from Ca²⁺, such as NaCl-containing solution, may be further free from any other divalent ions, including Mg²⁺. The NaCl-containing solution may be normal saline, which is the commonly used phrase for a solution of 0.90% w/v of NaCl, about 300 mOsm/L or 9.0 g per liter. Phosphate-buffered saline (abbreviated PBS), for example, contains sodium chloride, sodium phosphate, potassium phosphate and, in some formulations, potassium chloride, magnesium and calcium.

Alternatively, the pellets or particles, left in the second container in the step S5, may be re-suspended in the above-mentioned bottom portion of the centrifuged liquid remaining in the second container into a stem-cell containing solution (or called a stem-cell mixture), which thereby contains the pellets or particles. In this case, the step of adding the buffer or liquid into the second container as illustrated in the step S6 may be omitted.

In the present disclosure, the stem-cell containing solution may have a concentration of 10⁶ to 10⁷ SB cells (i.e., SB-1 cells and SB-2 cells) per milliliter. In addition, according to the results from Figs. 6A-6C and 7A-7G, it can be estimated, assumed or presumed that the percentage of SB-1 cells in cells greater than 2 micrometers and less than 6 micrometers in size in the stem-cell containing solution may be greater than 4% or 5%, and more preferably between 4.5% and 10%; the percentage of SB-2 cells in cells greater than 2 micrometers and less than 6 micrometers in size in the stem-cell containing solution may be greater than 4% or 5%, and more preferably between 4.5% and 10%; the percentage of BLSCs in cells less than 6 micrometers and greater than 2 micrometers in size in the stem-cell containing solution may be less than 6%, and more preferably less than 5% or 4.5%; the percentage of VSELs in cells less than 6 micrometers and greater than 2 micrometers in size in the stem-cell containing solution may be less than 2%, and more preferably less than 1% or 0.5%; less than 1% of cells in the stem-cell containing solution may express CD133; the percentage of platelets in cells less than 6 micrometers and greater than 2 micrometers in size in the stem-cell containing solution may be greater than 75% or 80%, and more preferably between 75% and 85%; the number of white blood cells per milliliter of the stem-cell containing solution may be less than 10⁴ or 10³, and more preferably less than 10²; the percentage of white blood cells in cells greater than 2 micrometers and less than 20 micrometers in size in the stem-cell containing solution may be less than 2%, and more preferably less than 1%, 0.5% or 0.1%; the number of red blood cells per milliliter of the stem-cell containing solution may be less than 10⁵ or 10⁴, and more preferably less than 10³; the percentage of red blood cells in cells greater than 2 micrometers and less than 10 micrometers in size in the stem-cell containing solution may be less than 3%, and more preferably less than 2%, 1% or 0.5%. Alternatively, the stem-cell containing solution may further contain the divalent cation chelating agent (e.g., EDTA) and/or growth factors.

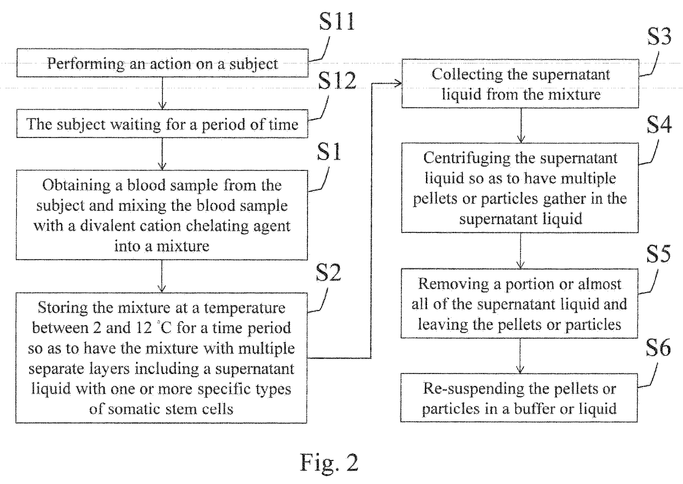

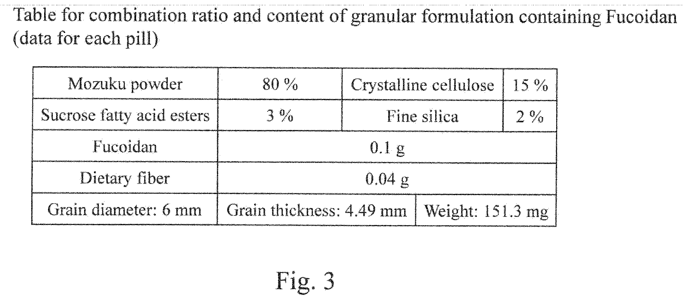

In an alternative embodiment, referring to Fig. 2, steps S11 and S12 may be performed before the step S1. In the step S11, the subject takes or is subjected to an action, which may be one of the above-mentioned actions (X). For example, in the step S11, the subject takes or ingests a mobilization agent such as a fucoidan-containing compound. The fucoidan-containing compound, for example, is a brown algae supplement. Fig. 3 shows the content/ingredient information of the brown algae supplement. In Fig. 3, it shows that a pill of the brown algae supplement contains 80% of a mozuku powder, 15% of crystalline cellulose, 3% of sucrose fatty acid esters, and 2% of micro or fine silica (containing silicon dioxide). The mozuku powder may be extracted from mozuku brown algae (one kind of seaweed) grown in the sea around and near Okinawa, Japan. The mozuku powder is then mixed with crystalline cellulose, sucrose fatty acid esters, and micro or fine silica (containing silicon dioxide) to form the pill of the brown algae supplement, which contains 0.1 grams of fucoidan. In the step S11, the subject may take or ingest 20 or more pills (e.g., at least 30 pills) of the brown algae supplement, which means the subject may take or ingest 2 grams or more (such as at least 3 grams) of fucoidan. In another example, in the step S11, the subject may be injected with a granulocyte-colony stimulating factor (GCSF), i.e., a mobilization agent, or may be subjected to a course of GCSF injections.

In the step S12, after the action in the step S11 is performed on the subject, the subject waits for a (predetermined) period of time, such as between 15 minutes and 60 minutes, between 20 minutes and 100 minutes, between 30 minutes and 4 hours, between 60 minutes and 90 minutes, between 0.5 hours and 3 hours, between 1 hour and 6 hours, between 1 hour and 12 hours, between 12 hours and 36 hours, or between 36 hours and 50 hours. With the steps S11 and S12, the one or more specific types of somatic stem cells (e.g., SB cells) can be mobilized into the peripheral blood of the subject from, e.g., the bone marrow of the subject; therefore, the peripheral blood of the subject becomes enriched with the one or more specific types of somatic stem cells. After the step S12 is performed, the steps S1 through S6 illustrated in Fig. 1 are sequentially performed so that the stem-cell containing solution is obtained or formed.

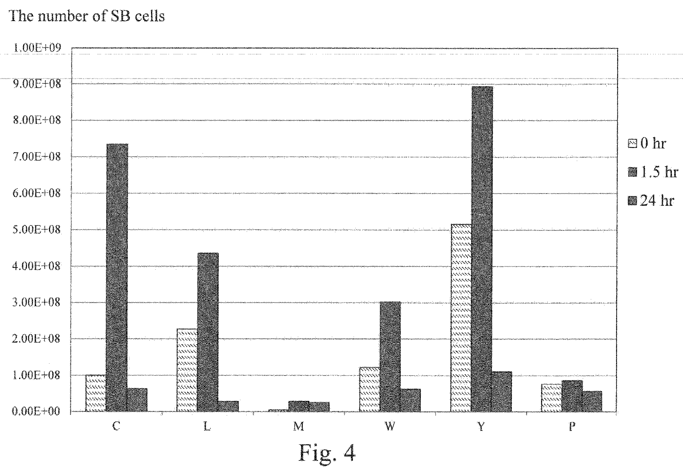

Fig. 4 is a graph presenting SB-cell data of six human subjects C, L, M, W, Y, and P obtained from peripheral-blood samples of the human subjects C, L, M, W, Y, and P in an experiment. In this experiment, the five human subjects C, L, M, W, and Y each orally ingested 20 pills of the brown algae supplement described in Fig. 3. In other words, each of the human subjects C, L, M, W, and Y orally ingested at least 2 grams of fucoidan. The human subject P was selected as a control in the experiment and did not ingest the brown algae supplement. In Fig. 4, 0 hr represents "before the ingestion of fucoidan" for the human subjects C, L, M, W, and Y and also represents "at the beginning of a control test" for the human subject P; 1.5 hr represents "at 1.5 hours after the ingestion of fucoidan" for the human subjects C, L, M, W, and Y and also represents "at 1.5 hours after the beginning of the control test" for the human subject P; 24 hr represents "at 24 hours after the ingestion of fucoidan" for the human subjects C, L, M, W, and Y and also represents "at 24 hours after the beginning of the control test" for the human subject P. As shown in Fig. 4, a significant increase in the number of CD349(+) cells and Lgr5(+) cells (i.e., SB cells) was found at 1.5 hours after the ingestion of the brown algae supplement, but an insignificant increase in the number of CD349(+) cells and Lgr5(+) cells was found at 1.5 hours after the beginning of the control test. Therefore, ingesting fucoidan, e.g., the brown algae supplement, can mobilize SB cells (e.g., CD349(+) cells and Lgr5(+) cells) into the peripheral blood (or the bloodstream) and enrich the peripheral blood with SB cells.

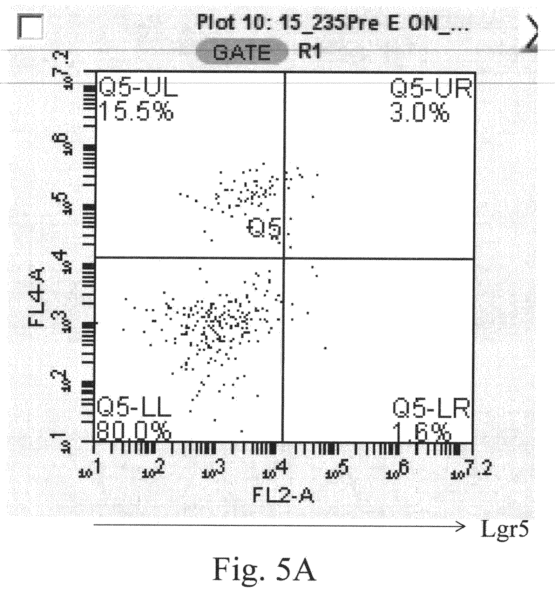

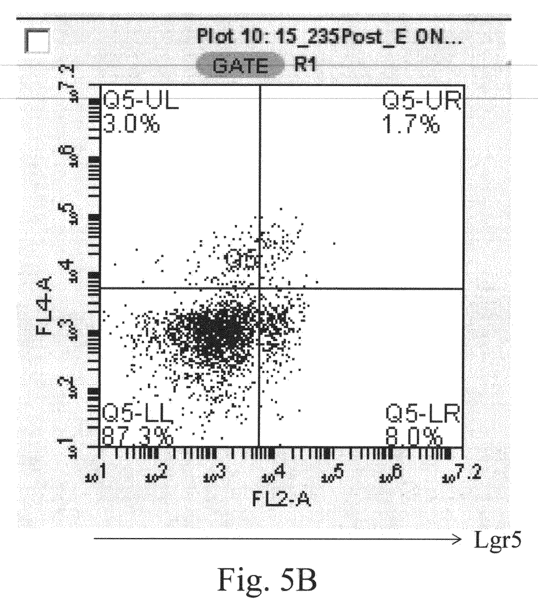

Fig. 5A shows flow cytometry data related to Lgr5(+) cells, i.e., SB2 cells, in a first peripheral-blood sample obtained from a human subject before a course of GCSF injections at a dose of 5 micrograms/kg/day for 5 consecutive days. Fig. 5B shows flow cytometry data related to Lgr5(+) cells in a second peripheral-blood sample obtained from the human subject after the course of GCSF injections. In this experiment, the human subject was subjected to 5 micrograms/kg per day of GCSF as a single injection for five consecutive days. The second peripheral-blood sample was collected approximately 3.5 hours after the last injection of GCSF. The results or data of Figs. 5A and 5B were obtained from a flow cytometer. The black points in the regions Q5-LR of Figs. 5A and 5B represent Lgr5(+) cells. With respect to the first peripheral-blood sample, the percentage of the number of Lgr5(+) cells in the region Q5-LR of Fig. 5A to the number of all particles or cells in the regions Q5-UL, Q5-UR, Q5-LL, and Q5-LR of Fig. 5A was 1.6%. With respect to the second peripheral-blood sample, the percentage of the number of Lgr5(+) cells in the region Q5-LR of Fig. 5B to the number of all particles or cells in the regions Q5-UL, Q5-UR, Q5-LL, and Q5-LR of Fig. 5B was 8%. Therefore, injecting GCSF can mobilize SB cells (especially Lgr5(+) cells) into the peripheral blood (or the bloodstream) and enrich the peripheral blood with SB cells.

It can be seen from the above results or data that the action in the step S11, such as ingesting the brown algae supplement or receiving a course of GCSF injections, may cause the one or more specific types of somatic stem cells (e.g., SB cells) mobilized into the peripheral blood of the subject to increase at a time point, e.g., of 1.5 hours or between 1 hour and 6 hours after the subject having taken or been subjected to the action. Therefore, the steps S11 and S12 may be performed to enrich the one or more specific types of somatic stem cells, such as SB cells (i.e., SB-1 cells and SB-2 cells), in the subject's peripheral blood.

In one embodiment, the stem-cell containing solution prepared, obtained or formed by performing the steps S1 through S6 illustrated in Fig. 1 or the steps S11, S12 and S1 through S6 illustrated in Fig. 2 may be employed or used for a bone graft procedure. The bone graft procedure, which may be used to rebuild lost tissue or augment insufficient tissue, includes mixing a (bone) graft material with the stem-cell containing solution so as to form or produce a second mixture of the (bone) graft material and the stem-cell containing solution, and administering to the subject or another subject (e.g., the alveolar bone of the subject or the other subject) in need thereof an effective amount of the second mixture. The other subject may have the same species as the subject. Alternatively, the other subject may have a different species from the subject. For example, the subject may be a mammal (e.g., pig), but the other subject may be a human. The (bone) graft material may be a synthetic bone material, a processed donor material, or autologous bone from the subject's own body. The bone graft procedure may be used for a bone- or joint-related treatment, such as a dental implant treatment. In the case of the bone graft procedure applied to a dental implant treatment, the bone graft procedure may be performed before the placement of a dental implant in the alveolar bone of the subject or the other subject. The second mixture in the alveolar bone of the subject or the other subject may stimulate bone development and create a dense, sturdy foundation for the dental implant.

In another embodiment, the stem-cell containing solution prepared, obtained or formed by performing the steps S 1 through S6 illustrated in Fig. 1 or the steps S11, S12 and S1 through S6 illustrated in Fig. 2 may be employed or used for a dental implant treatment. The dental implant treatment includes the following steps: processing a dental implant and then screwing the dental implant into the alveolar bone of the subject or another subject, wherein the step of processing the dental implant includes forming the stem-cell containing solution on a porous titanium-oxide layer on an outer thread (or a helix) of the dental implant by immersing the dental implant in the stem-cell containing solution or coating or dropping the stem-cell containing solution on or onto the porous titanium-oxide layer. The other subject may have the same species as the subject. Alternatively, the other subject may have a different species from the subject. For example, the subject may be a mammal (e.g., pig), but the other subject may be a human. The porous titanium-oxide layer may have micrometer pores, sub-micrometer pores and nanometer pores therein and may have a roughness greater than 100, 200 or 300 nm, such as between 200 and 700 nm or between 300 and 500 nm. After a period of time when the dental implant is screwed into the alveolar bone of the subject or the other subject, the stem-cell containing solution may enhance formation of a porous structure of nano-holes and micro-holes between the porous titanium-oxide layer of the dental implant and the alveolar bone of the subject or the other subject. For more elaboration, the porous titanium-oxide layer may be formed on a titanium surface of the outer thread of the dental implant.

In another embodiment, the stem-cell containing solution prepared, obtained or formed by performing the steps S 1 through S6 illustrated in Fig. 1 or the steps S11, S12 and S1 through S6 illustrated in Fig. 2 may be employed or used for a dental implant treatment. The dental implant treatment includes the following steps: processing a dental implant and then screwing the dental implant into the alveolar bone of the subject or another subject, wherein the step of processing the dental implant includes forming the stem-cell containing solution on a titanium surface of an outer thread (or a helix) of the dental implant by immersing the dental implant in the stem-cell containing solution or coating or dropping the stem-cell containing solution on or onto the titanium surface of the outer thread of the dental implant. The other subject may have the same species as the subject. Alternatively, the other subject may have a different species from the subject. For example, the subject may be a mammal (e.g., pig), but the other subject may be a human. The titanium surface of the outer thread of the dental implant may have a roughness less than 50 or 100 nm, such as between 20 and 80 nm or between 30 and 45 nm. After a period of time when the dental implant is screwed into the alveolar bone of the subject or the other subject, the stem-cell containing solution may cause a porous structure of nano-holes and micro-holes formed between the titanium surface of the outer thread of the dental implant and the alveolar bone of the subject or the other subject.

In another embodiment, the stem-cell containing solution prepared, obtained or formed by performing the steps S 1 through S6 illustrated in Fig. 1 or the steps S11, S12 and S1 through S6 illustrated in Fig. 2 may be employed or used for a dental implant treatment. The dental implant treatment includes screwing a dental implant with a porous titanium-oxide layer on an outer thread (or a helix) thereof into the alveolar bone of the subject or another subject, and then coating or dropping the stem-cell containing solution on or onto an exposed portion, i.e., a top portion, of the dental implant such that the stem-cell containing solution flows along the interface between the porous titanium-oxide layer of the dental implant and the alveolar bone of the subject or the other subject to form the stem-cell containing solution between the porous titanium-oxide layer of the dental implant and the alveolar bone of the subject or the other subject. The other subject may have the same species as the subject. Alternatively, the other subject may have a different species from the subject. For example, the subject may be a mammal (e.g., pig), but the other subject may be a human. The porous titanium-oxide layer may have micrometer pores, sub-micrometer pores and nanometer pores therein and may have a roughness greater than 100, 200 or 300 nm, such as between 200 and 700 nm or between 300 and 500 nm. After a period of time when the stem-cell containing solution is formed between the porous titanium-oxide layer of the dental implant and the alveolar bone of the subject or the other subject, the stem-cell containing solution may enhance formation of a porous structure of nano-holes and micro-holes between the porous titanium-oxide layer of the dental implant and the alveolar bone of the subject or the other subject. For more elaboration, the porous titanium-oxide layer may be formed on a titanium surface of the outer thread of the dental implant.

In another embodiment, the stem-cell containing solution prepared, obtained or formed by performing the steps S 1 through S6 illustrated in Fig. 1 or the steps S11, S12 and S1 through S6 illustrated in Fig. 2 may be employed or used for a dental implant treatment. The dental implant treatment includes screwing a dental implant having an outer thread (or a helix) with a titanium surface into the alveolar bone of the subject or another subject, and then coating or dropping the stem-cell containing solution on or onto an exposed portion, i.e., a top portion, of the dental implant such that the stem-cell containing solution flows along the interface between the titanium surface of the outer thread of the dental implant and the alveolar bone of the subject or the other subject to form the stem-cell containing solution between the titanium surface of the outer thread of the dental implant and the alveolar bone of the subject or the other subject. The other subject may have the same species as the subject. Alternatively, the other subject may have a different species from the subject. For example, the subject may be a mammal (e.g., pig), but the other subject may be a human. The titanium surface of the outer thread of the dental implant may have a roughness less than 50 or 100 nm, such as between 20 and 80 nm or between 30 and 45 nm. After a period of time when the stem-cell containing solution is formed between the titanium surface of the outer thread of the dental implant and the alveolar bone of the subject or the other subject, the stem-cell containing solution may cause a porous structure of nano-holes and micro-holes formed between the titanium surface of the outer thread of the dental implant and the alveolar bone of the subject or the other subject.

In another embodiment, the stem-cell containing solution prepared, obtained or formed by performing the steps S 1 through S6 illustrated in Fig. 1 or the steps S11, S12 and S1 through S6 illustrated in Fig. 2 may be employed or used for treating joints, (muscle) tendon, (knee) articular cartilage, shoulder, or spine. The treatment includes the following steps: performing the steps S1 through S6 illustrated in Fig. 1 or the steps S11, S12, and S1 through S6 illustrated in Fig. 2 to prepare, obtain or form the stem-cell containing solution, and then injecting an effective amount of the stem-cell containing solution into the subject or another subject, such as joints, (muscle) tendon, (knee) articular cartilage, shoulder, spine, or muscle of the subject or the other subject. The other subject may have the same species as the subject. Alternatively, the other subject may have a different species from the subject. For example, the subject may be a mammal (e.g., pig), but the other subject may be a human.

In another embodiment, the stem-cell containing solution prepared, obtained or formed by performing the steps S 1 through S6 illustrated in Fig. 1 or the steps S11, S12 and S1 through S6 illustrated in Fig. 2 may be employed or used for a treatment for arthritis (e.g., osteoarthritis, psoriatic arthritis, rheumatoid arthritis, ankylosing spondylitis), tendonitis, tendon injury, or an autoimmune disease or disorder (e.g., rheumatoid arthritis, ankylosing spondylitis, or systemic lupus erythematosus). The treatment includes the following steps: performing the steps S1 through S6 illustrated in Fig. 1 or the steps S11, S12 and S1 through S6 illustrated in Fig. 2 to obtain or form the stem-cell containing solution, and then injecting an effective amount of the stem-cell containing solution into the subject or another subject, such as joints, (muscle) tendon, (knee) articular cartilage, shoulder, spine, or muscle of the subject or the other subject. The other subject may have the same species as the subject. Alternatively, the other subject may have a different species from the subject. For example, the subject may be a mammal (e.g., pig), but the other subject may be a human.

All of the features disclosed in this specification may be combined in any combination. Each feature disclosed in this specification may be replaced by an alternative feature serving the same, equivalent, or similar purpose. Thus, unless expressly stated otherwise, each feature disclosed is only an example of a generic series of equivalent or similar features.

A number of embodiments have been described. Nevertheless, it will be understood that various modifications may be made without departing from the spirit and scope of the invention. Accordingly, other embodiments are within the scope of the following claims.

## Claims

1. A method of preparing a stem-cell containing solution, comprising:
storing a mixture of a blood sample and a divalent cation chelating agent at a temperature between 2 and 12 degrees Celsius for a time period of from 3 hours to 72 hours so as to have said mixture with multiple separate layers, wherein a first liquid in a first one of said separate layers comprises multiple somatic stem cells and multiple platelets;
collecting said first liquid; and
after said collecting said first liquid, centrifuging said first liquid so as to have multiple particles gather in said first liquid, wherein said particles comprise said somatic stem cells and said platelets.

2. The method of Claim 1, wherein said divalent cation chelating agent comprises ethylenediaminetetraacetic acid (EDTA) or citrate.

3. The method of Claim 1 or 2, wherein said temperature is between 2 and 7 degrees Celsius.

4. The method of any one of Claims 1 to 3, wherein said time period is from 16 hours to 72 hours or from 36 hours to 60 hours.

5. The method of any one of Claims 1 to 4, wherein said somatic stem cells are between 1 and 6 micrometers in size and comprise CD349(+) stem cells and Lgr5(+) stem cells.

6. The method of any one of Claims 1 to 5, wherein the percentage of white blood cells in cells greater than 2 micrometers and less than 20 micrometers in size in said particles is less than 2%.

7. The method of any one of Claims 1 to 6, after said centrifuging said first liquid, further comprising removing a first portion of said first liquid and leaving said particles gathering in a second portion of said first liquid, and then re-suspending said particles in said second portion of said first liquid.

8. The method of any one of Claims 1 to 6, after said centrifuging said first liquid, further comprising removing substantially all or a first portion of said first liquid and leaving said particles, and then re-suspending said particles in a second liquid.

9. The method of Claim 8, wherein said second liquid comprises a salt solution.

10. The method of Claim 8 or 9, wherein said second liquid comprises a second portion of said first liquid.

11. The method of any one of Claims 1 to 10, wherein said first liquid further comprises said divalent cation chelating agent.

12. The method of any one of Claims 1 to 11, wherein a second one of said separate layers comprises at least 98 percent of red blood cells in said mixture and at least 98 percent of white blood cells in said mixture.

13. The method of any one of Claims 1 to 12, wherein said blood sample is obtained from a subject immediately after ingesting fucoidan for a period of time between 30 minutes and 4 hours.

14. The method of any one of Claims 1 to 13, wherein said stem-cell containing solution is used for a dental implant treatment.

15. The method of any one of Claims 1 to 13, wherein said stem-cell containing solution is used for a bone graft procedure.
